# EUROPÄISCHE PATENTSCHRIFT

(11) **EP 2 322 131 B1**
(45) Veröffentlichungstag und Bekanntmachung des Hinweises auf die Patenterteilung: **16.07.2014**
(21) Anmeldenummer: 10190710.3
(22) Anmeldetag: 10.11.2010
(51) Int. Cl.: A61H 33/06

(54) **Infrarotwärmekabine**
Infrared heating cabin
Cabine de chauffage infrarouge

(30) Priorität: 11.11.2009 AT 7102009
(43) Veröffentlichungstag der Anmeldung: 18.05.2011
(73) Patentinhaber: Lindner, Heide, 5310 Mondsee (AT)
(72) Erfinder: Lindner, Heide, 5310 Mondsee (AT)
(74) Vertreter: KLIMENT & HENHAPEL

(56) Entgegenhaltungen:
- DE-C1- 19 546 392
- DE-U1- 20 116 988
- DE-U1-202004 010 180
- NL-C2- 1 021 063
- US-A- 3 624 844
- US-A1- 2007 089 229

## Beschreibung

Die Erfindung bezieht sich auf eine Wärmebehandlungskabine, umfassend einen von Seitenwänden, einer Decke, einem Boden und mindestens einer Tür begrenzten Raum, innerhalb welchem ein Sitzaufbau und eine beliebige Anzahl an Wärmestrahlelementen angeordnet ist, gemäß dem Oberbegriff des Anspruchs 1.

Wärmebehandlungskabinen erfreuen sich im Gesundheits- und Wellness-Bereich großer Beliebtheit und dienen dazu, präventiv oder therapeutisch auf den menschlichen Körper einzuwirken, indem der Stoffwechsel und das Immunsystem durch technisch bereitgestellte Wärme stimuliert wird.

Zur Bereitstellung dieser innerhalb der Wärmebehandlungskabine zu erzeugenden Wärme dienen neben klassischen Saunaöfen auch elektrisch betriebene Wärmestrahlelemente. Das Strahlungsspektrum derartiger Wärmestrahlelemente liegt hauptsächlich im Infrarot-Bereich, sodass man in diesem Zusammenhang auch von Infrarot-Strahlern spricht.

Gegenüber klassischen Saunaöfen, welche die Luft innerhalb der Wärmebehandlungskabine hauptsächlich mittels Konvektionswärme aufheizen, ergibt sich bei Wärmestrahlelementen ein wesentlich angenehmeres Klima. Unter Einsatz von Wärmestrahlelementen ist die Lufttemperatur deutlich niedriger als unter Einsatz von klassischen (Konvektions-)Saunaöfen, wobei dennoch eine tiefere Einwirkung der emittierten Wärme in das Gewebe des menschlichen Körpers erfolgt.

Als nachteilig erweist sich bei bekannten Wärmebehandlungskabinen jedoch die konventionelle Anordnung der Wärmestrahlelemente, mit welcher spezielle, eventuell therapiebedürftige Körperpartien einer auf dem Sitzaufbau sitzenden Person keiner ausreichenden Wärmeeinwirkung unterzogen werden können.

Bei bekannten Wärmebehandlungskabinen sind zumeist 2-5 rechteckige bzw. paneelartige Wärmestrahlelemente in jeweils festgelegter Position an den Innenwänden der Wärmebehandlungskabine angebracht, z.B. angeschraubt.

Hierbei werden nur jene den wandseitigen Wärmestrahlelementen zugewandten Bereiche des Körpers effektiv bestrahlt, während jene von den Wärmestrahlelementen abgewandten Bereich des Körpers, insbesondere dem Becken-, Rumpf- und dem hinteren Oberschenkelbereich nur eine indirekte und daher relativ geringe Wärmeeinwirkung zuteil wird.

Der vorliegenden Erfindung liegt daher die Aufgabe zugrunde, eine Wärmebehandlungskabine samt Wärmestrahlelementen bereitzustellen, mittels welchen eine fokussierte Wärmebestrahlung insbesondere des Becken-, Rumpf- und des hinteren Oberschenkelbereichs einer auf dem Sitzaufbau sitzenden Person ermöglicht wird.

Diese Aufgabe wird durch eine Wärmebehandlungskabine mit den kennzeichnenden Merkmalen des Anspruchs 1 gelöst.

Bei einer gattungsgemäßen Wärmebehandlungskabine, umfassend einen von Seitenwänden, einer Decke, einem Boden und mindestens einer Tür begrenzten Raum, innerhalb welchem ein Sitzaufbau und eine beliebige Anzahl an Wärmestrahlelementen angeordnet ist, ist es erfindungsgemäß vorgesehen, dass die Wärmestrahlelemente als elektrisch betriebene Infrarot-Strahler ausgeführt sind, und zumindest einer der elektrisch betriebenen Infrarot-Strahler unmittelbar unterhalb einer Sitzfläche des Sitzaufbaus im Sitzaufbau versenkt angeordnet ist und von einem die Sitzfläche zumindest abschnittsweise ausbildenden Abdeckelement abgedeckt ist, wobei die hauptsächliche Wärmestrahlrichtung des elektrisch betriebene Infrarot-Strahlers im Wesentlichen orthogonal zur Sitzfläche des Sitzaufbaus verläuft und das Abdeckelement aus Plexiglas ausgebildet ist.

Auf diese Weise können der Becken-, Rumpf- und der hintere Oberschenkelbereich, eventuell auch der Wadenbereich des menschlichen Körpers gezielt mit Wärme bestrahlt werden.

In der Fachsprache bedeutet "versenkt anordnen", etwas so anzubringen, dass es nicht übersteht oder herausragt: so werden etwa Nägel, Nieten oder Schrauben versenkt angeordnet. Der versenkte Gegenstand kann höchstens mit der Oberfläche eine Ebene bilden.

Im gegenständlichen Fall ersetzt also zumindest ein Wärmestrahlelement einen Teil des Sitzaufbaus.

Dies wurde im Stand der Technik, welcher der Anmelderin bekannt ist, bisher anders gelöst, wobei etwa die DE 20 2006 008 274 U1 eine durchgängige Sitzbank zeigt, unterhalb derer ein Wärmestrahler angeordnet ist. Dieser ist aber nicht Teil der Sitzfläche oder der Sitzbank und damit nicht Teil des Sitzaufbaus. Ebenso ist die Sitzbank in der DE 201 16 988 U1 als ganzes aus einem für Infrarotwärmestrahlen durchlässigen Material hergestellt, und unterhalb der Sitzbank ein Bodenstrahler angeordnet.

### Auch aus der DE 195 46 392 C1 ist es bekannt, einen Wärmestrahler unterhalb der Sitzbank anzuordnen. Des weiteren ist es bekannt einen als Stein ausgebildeten Wärmestrahler, hinter einer Sitzfläche einer Sitzbank, bündig mit der Sitzfläche anzuordnen.

Ein weiteres Beispiel einer Wärmekabine ist in US 2007/0089229 beschrieben. Bei einer Ausführungsform der Erfindung ist die Sitzfläche vom Boden abgewandt und vorzugsweise horizontal angeordnet.

Eine besonders wirksame Wärmeeinwirkung auf den menschlichen Körper wird erzielt, indem die Wärmestrahlrichtung des Wärmestrahlelementes vorzugsweise im Wesentlichen in Richtung der Decke der Wärmebehandlungskabine verläuft.

Die Wärmestrahlrichtung des Wärmestrahlelementes verläuft hierbei im Wesentlichen orthogonal zur Sitzfläche des Sitzaufbaus. Indem also die hauptsächliche Wärmemenge vertikal nach oben emittiert wird, kann eine gezielte Durchwärmung des Becken-, Rumpf- sowie des hinteren Oberschenkelbereiches einer auf der Sitzfläche des Sitzaufbaus sitzenden Person erfolgen.

Das im Sitzaufbau versenkte Wärmestrahlelement ist von einem die Sitzfläche zumindest abschnittsweise ausbildenden Abdeckelement abgedeckt. Das Abdeckelement ist aus Glas ausgebildet.

Gemäß einer speziellen Ausführungsvariante der Erfindung ist ein weiteres, im Sitzaufbau versenktes Wärmestrahlelement hinter einer vorzugsweise vertikalen Seitenfläche des Sitzaufbaus angeordnet. Auf diese Weise können auch der Kniekehlen- und der Wadenbereich einer auf dem Sitzaufbau sitzenden Person wirksam mit Wärme bestrahlt werden.

Die Erfindung wird nun anhand eines Ausführungsbeispiels näher erläutert. Dabei zeigt:
- Fig.1: eine schematische Darstellung einer erfindungsgemäßen Wärmebehandlungskabine in Isometrieansicht
- Fig.2: eine Detailansicht eines in erfindungsgemäßer Weise mit einem Wärmestrahlelement bestückten Sitzaufbaus der Wärmebehandlungskabine
- Fig.3: eine alternative Ausführungsform eines erfindungsgemäßen Sitzaufbaus der Wärmebehandlungskabine
- Fig.4: eine schematische Darstellung einer erfindungsgemäßen Wärmebehandlungskabine in vertikaler Schnittansicht

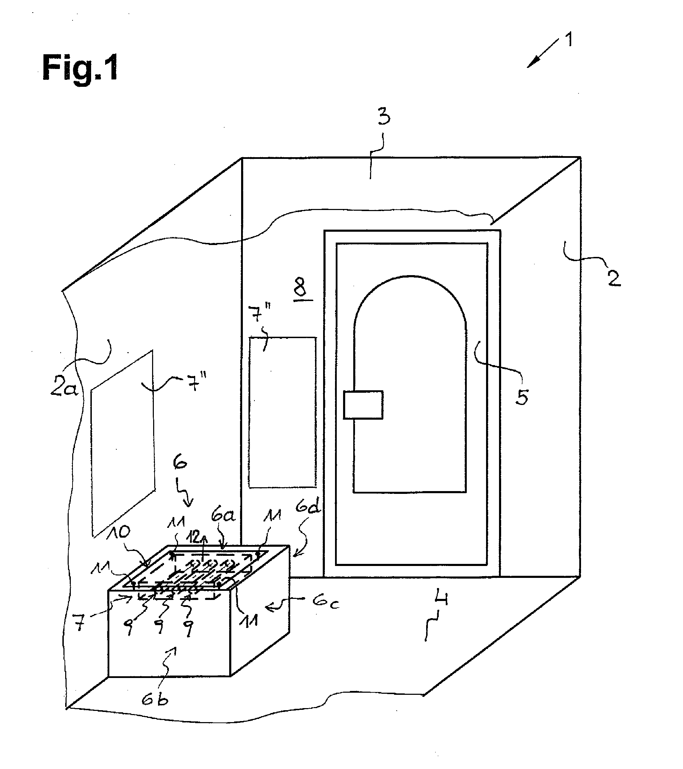

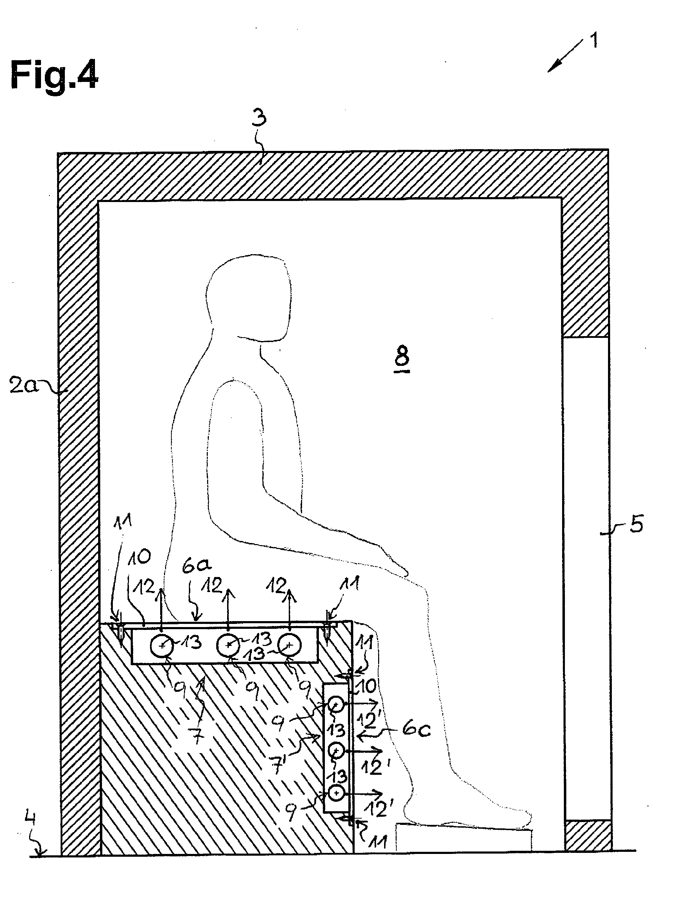

Fig.1 zeigt einen Abschnitt einer einfachen quaderförmigen Wärmebehandlungskabine 1, bei welcher mittels Seitenwänden 2, einer Decke 3, einem Boden 4 und einer Tür 5 ein Raum 8 konstituiert wird, innerhalb welchem sich ein schematisch dargestellter Sitzaufbau 6 befindet. Wie in einer Seitenansicht der Wärmebehandlungskabine 1 gemäß Fig.4 ersichtlich, grenzt der üblicherweise aus Holz ausgeführte Sitzaufbau 6 an eine der Tür 5 gegenüberliegende Seitenwand 2 bzw. an eine Rückwand 2a der Wärmebehandlungskabine 1 an.

Erfindungsgemäß ist zumindest ein Wärmestrahlelement 7 im Sitzaufbau 6 versenkt angeordnet.

Die Wärmestrahlelemente 7 können eine beliebige Geometrie aufweisen und sowohl ebene als auch gekrümmte oder gewinkelte Strahlungsflächen mit oder ohne Reflektoren aufweisen.

Im Ausführungsbeispiel gemäß Fig.1 ist das Wärmestrahlelement 7 hinter, d.h. unterhalb einer vom Boden 4 abgewandten, vorzugsweise horizontalen Sitzfläche 6a des Sitzaufbaus 6 angeordnet, indem es in eine entsprechende - hier nach oben offene - Vertiefung des Sitzaufbaus 6 versenkt wird, die sich unmittelbar unterhalb der Sitzfläche 6a befindet. Das Wärmestrahlelement 7 ist hierbei zu den Seitenwänden 2, insbesondere zur Rückwand 2a der Wärmebehandlungskabine 1 beabstandet angeordnet.

Gemäß dem vorliegenden Ausführungsbeispiel umfasst das erfindungsgemäß angeordnete Wärmestrahlelement 7 mehrere im Wesentlichen in einer im Wesentlichen horizontalen Ebene verlaufende Infrarot-Röhren 9. Das Wärmestrahlelement 7 könnte auch gewendelt oder paneelförmig ausgebildet sein.

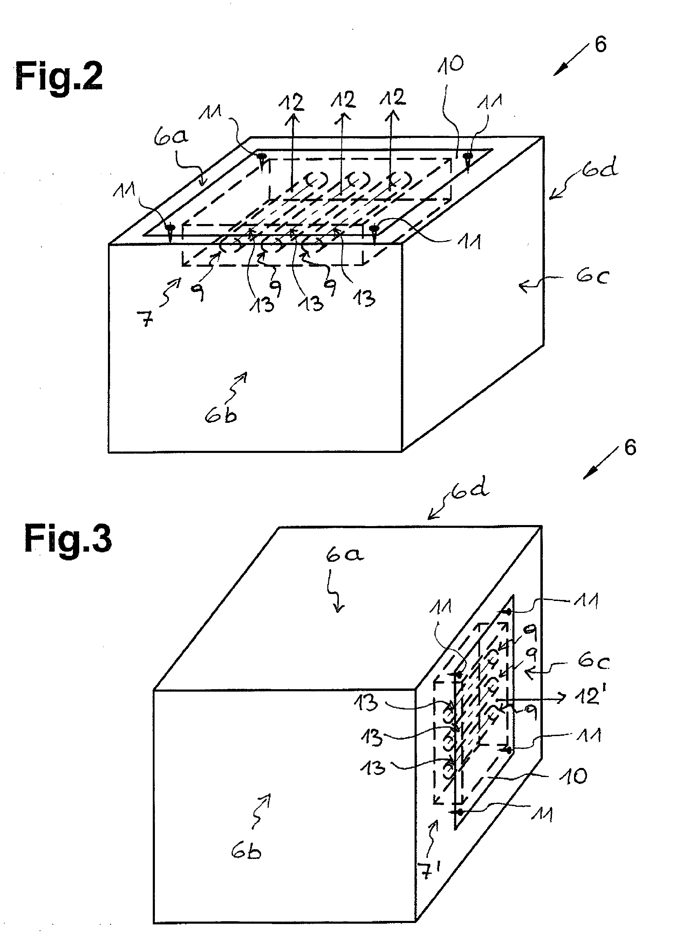

Wie in Fig.2 detailliert ersichtlich, verlaufen die Längsachsen 13 der Infrarotröhren 9 in einer im Wesentlichen horizontalen Richtung bzw. im Wesentlichen parallel zur Sitzfläche 6a des Sitzaufbaus 6.

Die mit Pfeil 12 angedeutete (hauptsächliche) Wärmestrahlrichtung des Wärmestrahlelementes 7 verläuft im Wesentlichen in Richtung der Decke 3 der Wärmebehandlungskabine 1. Die Wärmestrahlrichtung 12 des Wärmestrahlelementes 7 wird jeweils durch die Anordnung der Infrarot-Röhren 9 sowie nicht dargestellter, an einer dem Boden 4 zugewandten Unterseite des Wärmestrahlelementes 7 angeordneter Reflektoren festgelegt.

Die Wärmestrahlrichtung 12 des Wärmestrahlelementes 7 verläuft hierbei im Wesentlichen orthogonal zur Sitzfläche 6a des Sitzaufbaus 6.

Wie ebenfalls in Fig.2 ersichtlich, ist das im Sitzaufbau 6 versenkte Wärmestrahlelement 7 von einem Abdeckelement 10 abgedeckt. Das aus Glas ausgebildete Abdeckelement 10 ist mittels z.B. als Schrauben ausgebildeter Befestigungselemente 11 am Sitzaufbau 6 befestigt und kann die gesamte Sitzfläche 6a oder zumindest einen Abschnitt der Sitzfläche 6a ausbilden.

Ergänzend zum vorangehend beschriebenen und in den Figuren 1 und 2 dargestellten Wärmestrahlelement 7, kann gemäß Fig.3 ein weiteres, im Sitzaufbau 6 versenktes Wärmestrahlelement 7' vorgesehen sein, welches hinter einer vorzugsweise vertikalen Seitenfläche 6b, 6c, 6d des Sitzaufbaus 6 angeordnet ist. Vorzugsweise ist das weitere Wärmestrahlelement 7' hierbei hinter einer von der Rückwand 2a abweisenden vorderen Seitenfläche 6c des Sitzaufbaus 6, also in jenem Bereich des Sitzaufbaus 6, welcher zu den Unterschenkel- bzw. Wadenbereich einer auf dem Sitzaufbau 6 sitzenden Person gerichtet ist.

Die mit Pfeil 12' angedeutete (hauptsächliche) Wärmestrahlrichtung des weiteren Wärmestrahlelementes 7' verläuft gemäß Fig.3 im Wesentlichen horizontal bzw. orthogonal zur Seitenfläche 6c des Sitzaufbaus 6.

Zusätzlich zu den beschriebenen Wärmestrahlelementen 7 und 7' können an den Seitenwänden 2 oder an der Decke 3 noch beliebig viele konventionelle Wärmestrahlelemente 7" angeordnet sein (in Fig.1 schematisch angedeutet). Die in Fig.1 dargestellten konventionellen Wärmestrahlelemente 7" besitzen eine vorzugsweise im Wesentlichen vertikal verlaufende Strahlungsfläche.

Eine dem Raum 8 zugewandte Vorderseite des Abdeckelementes 10 verläuft vorzugsweise bündig mit der vom Sitzaufbau 6 ausgebildeten Sitzfläche 6a bzw. mit einer der Seitenflächen 6a des Sitzaufbaus 6, kann jedoch auch nach vorne oder nach hinten versetzt zu diesen angeordnet sein.

## Patentansprüche

1. Wärmebehandlungskabine (1), umfassend einen von Seitenwänden (2), einer Decke (3), einem Boden (4) und mindestens einer Tür (5) begrenzten Raum (8), innerhalb welchem ein Sitzaufbau (6) und eine beliebige Anzahl an Wärmestrahlelementen (7) angeordnet ist, **dadurch gekennzeichnet, dass** die Wärmestrahlelemente (7) als elektrisch betriebene Infrarot-Strahler ausgeführt sind, und zumindest einer der elektrisch betriebenen Infrarot-Strahler in einer sich unmittelbar unterhalb einer Sitzfläche (6a) des Sitzaufbaus (6) befindlichen, nach oben offenen Vertiefung im Sitzaufbau (6) versenkt angeordnet ist und von einem die Sitzfläche (6a) zumindest abschnittsweise ausbildenden Abdeckelement (10) abgedeckt ist, wobei die hauptsächliche Wärmestrahlrichtung (12) des elektrisch betriebenen Infrarot-Strahlers im Wesentlichen orthogonal zur Sitzfläche (6a) des Sitzaufbaus (6) verläuft und das Abdeckelement (10) aus Glas ausgebildet ist.

2. Wärmebehandlungskabine (1) nach Anspruch 1, **dadurch gekennzeichnet, dass** die Sitzfläche (6a) vom Boden (4) abgewandt und horizontal verlaufend angeordnet ist.

3. Wärmebehandlungskabine (1) nach Anspruch 2, **dadurch gekennzeichnet, dass** die Wärmestrahlrichtung (12) des Wärmestrahlelementes (7) im Wesentlichen in Richtung der Decke (3) der Wärmebehandlungskabine (1) verläuft.

4. Wärmebehandlungskabine (1) nach einem der Ansprüche Anspruch 1 bis 3, **dadurch gekennzeichnet, dass** ein weiteres, im Sitzaufbau (6) versenktes Wärmestrahlelement (7') hinter einer vorzugsweise vertikalen Seitenfläche (6b, 6c, 6d) des Sitzaufbaus (6) angeordnet ist.

## Claims

1. A heat treatment cabin (1) comprising a space (8) limited by side walls (2), a ceiling (3), a floor (4) and at least one door (5), which has a seat structure (6) and a random number of heat radiating elements (7) arranged inside it, **characterised in that** the heat radiating elements (7) are configured as electrically driven infrared radiators, and at least one of the electrically driven infrared radiators is arranged in a recess in the seat structure (6) directly below a seat surface (6a) of the seat structure (6) and open in upward direction and is covered by a covering element (10) forming the seat surface (6a) at least in sections, wherein the main heat radiating direction (12) of the electrically driven infrared radiator extends essentially orthogonally to the seat surface (6a) of the seat structure (6) and the covering element (10) is made of glass.

2. The heat treatment cabin (1) according to claim 1, **characterised in that** the seat surface (6a) is arranged facing away from the floor (4) and extending in horizontal direction.

3. The heat treatment cabin (1) according to claim 2, **characterised in that** the heat radiating direction (12) of the heat radiating element (7) essentially extends in direction of the ceiling (3) of the heat treatment cabin (1).

4. The heat treatment cabin (1) according to one of claims 1 to 3, **characterised in that** a further heat radiating element (7') recessed in the seat structure (6) is arranged behind a preferably vertical side surface (6b, 6c, 6d) of the seat structure (6).

## Revendications

1. Cabine de traitement thermique (1), comprenant une cellule (8) limitée par des parois latérales (2), un plafond (3), un sol (4) et au moins une porte (5) et à l'intérieur de laquelle une structure de siège (6) et un nombre quelconque d'éléments de rayonnement thermique (7) sont disposés, **caractérisée en ce que** les éléments de rayonnement thermique (7) se présentent sous forme de projecteurs infrarouges fonctionnant à l'électricité et qu'au moins un des projecteurs infrarouges fonctionnant à l'électricité est disposé enfoncé dans un creux de la structure de siège (6) ouvert vers le haut et se trouvant directement en-dessous d'une surface d'assise (6a) de la structure de siège (6) et est recouvert par un élément de recouvrement (10) constituant du moins par endroits la surface d'assise (6a), le sens principal de rayonnement thermique (12) du projecteur infrarouge fonctionnant à l'électricité étant orienté sensiblement orthogonalement par rapport à la surface d'assise (6a) de la structure de siège (6) et l'élément de recouvrement (10) étant en verre.

2. Cabine de traitement thermique (1) selon la revendication 1, **caractérisée en ce que** la surface d'assise (6a) est détournée du sol (4) et est disposée orientée horizontalement.

3. Cabine de traitement thermique (1) selon la revendication 2, **caractérisée en ce que** le sens de rayonnement thermique (12) de l'élément de rayonnement thermique (7) est orienté sensiblement en direction du plafond (3) de la cabine de traitement thermique (1).

4. Cabine de traitement thermique (1) selon une des revendications 1 à 3, **caractérisée en ce qu'**un autre élément de rayonnement thermique (7') enfoncé dans la structure de siège (6) est disposé derrière une surface latérale (6b, 6c, 6d) de préférence verticale de la structure de siège (6).
